# EUROPÄISCHE PATENTSCHRIFT

(11) **EP 1 599 144 B1**
(45) Veröffentlichungstag und Bekanntmachung des Hinweises auf die Patenterteilung: **28.07.2010**
(21) Anmeldenummer: 04710804.8
(22) Anmeldetag: 13.02.2004
(51) Int. Cl.: A61B 17/58

(54) **CHIRURGISCHES INSTRUMENT**
SURGICAL INSTRUMENT
INSTRUMENT CHIRURGICAL

(30) Priorität: 27.02.2003 DE 10310004
(43) Veröffentlichungstag der Anmeldung: 30.11.2005
(60) Teilanmeldung: 10158084.3
(73) Patentinhaber: Aesculap AG, 78532 Tuttlingen (DE)
(72) Erfinder: NESPER, Markus, 78532 Tuttlingen (DE); PLEIL, Thomas, 78073 Bad Dürrheim (DE); STEINHILPER, Klaus-Dieter, 78532 Tuttlingen (DE); WEISSHAUPT, Dieter, 78194 Immendingen (DE)
(74) Vertreter: Pioch, Holger
(86) Internationale Anmeldenummer: PCT/EP2004/001346
(87) Internationale Veröffentlichungsnummer: WO 2004/075765

(56) Entgegenhaltungen:
- EP-A- 0 857 466
- DE-C- 19 832 798
- DE-U- 29 812 988
- US-B1- 6 379 363

## Beschreibung

Die Erfindung betrifft ein chirurgisches Instrument zum Anlegen einer Knochenplatten-Fixiervorrichtung, welche ein erstes Knochenanlageelement mit einem von diesem abstehenden stabförmigen, eine Längsrichtung definierenden Verbindungsglied und ein auf dem Verbindungsglied in Richtung auf das erste Knochenanlageelement verschiebbares zweites Knochenanlageelement aufweist, mit einem an dem zweiten Knochenanlageelement in einer Anlagestellung anlegbaren ersten Werkzeugelement und einem von dem ersten Werkzeugelement entfernbaren zweiten Werkzeugelement, mit einer Transportvorrichtung zum schrittweisen Transportieren des Verbindungsglieds mit dem zweiten Werkzeugelement in mehreren Transportschritten in einer proximalen Richtung weg von dem in der Anlagestellung am zweiten Knochenanlageelement anliegenden ersten Werkzeugelement, wobei das zweite Werkzeugelement mehrere Aufnahmen für einen am Verbindungsglied abstehenden Vorsprung aufweist, wobei das zweite Werkzeugelement eine eine Mehrzahl von Zähnen umfassende Verzahnung aufweist und die Verzahnung die Aufnahmen umfasst, wobei der Vorsprung eine mindestens zwei Zähne umfassende Vorsprungverzahnung aufweist, bei jedem Transportschritt mindestens teilweise mit einer Aufnahme in einer Eingriffsposition in Eingriff bringbar und in dieser in Längsrichtung unbeweglich am zweiten Werkzeugelement gehalten ist und wobei von einem Transportschritt zu einem nachfolgenden Transportschritt der Vorsprung mit einer in proximalerer Richtung am zweiten Werkzeugelement angeordneten Aufnahme in Eingriff bringbar ist.

Ferner betrifft die vorliegende Erfindung eine Knochenplatten-Fixiervorrichtung, welche ein erstes Knochenanlageelement mit einem von diesem abstehenden stabförmigen, eine Längsrichtung definierenden Verbindungsglied und ein auf dem Verbindungsglied in Richtung auf das erste Knochenanlageelement verschiebbares zweites Knochenanlageelement aufweist, wobei das Verbindungsglied mit Rückhaltevorsprüngen versehen ist, wodurch eine Verschiebung des zweiten Anlageelements relativ zum ersten Anlageelement von diesem weg aufgrund der in diese Richtung wirkenden Rückhaltevorsprünge unmöglich ist, wobei das Verbindungsglied einen abstehenden Vorsprung aufweist, welcher eine mindestens zwei Zähne umfassende Vorsprungverzahnung aufweist und wobei die Knochenplatten-Fixiervorrichtung mit einem chirurgischen Instrument anlegbar ist, welches Instrument ein an dem zweiten Knochenanlageelement in einer Anlagestellung anlegbares erstes Werkzeugelement und ein von dem ersten Werkzeugelement entfernbares zweites Werkzeugelement, eine Transportvorrichtung zum schrittweisen Transportieren des Verbindungsglieds mit dem zweiten Werkzeugelement in mehreren Transportschritten in einer proximalen Richtung weg von dem in der Anlagestellung am zweiten Knochenanlageelement anliegenden ersten Werkzeugelement umfasst, wobei das zweite Werkzeugelement mehrere Aufnahmen für den Vorsprung aufweist, wobei das zweite Werkzeugelement eine eine Mehrzahl von Zähnen umfassende Verzahnung aufweist und die Verzahnung die Aufnahmen umfasst.

Ein Instrument zum Anlegen einer Knochenplatten-Fixiervorrichtung ist beispielsweise aus der DE 197 00 474 C2 bekannt. Mit einem von zwei Spannbacken gebildeten zweiten Werkzeugelement kann das stabförmige Verbindungsglied in einer Klemmstellung geklemmt und in der Klemmstellung relativ zum zweiten Knochenanlageelement bewegt werden. Ein Nachfassen des Verbindungsglieds mit den Spannbacken ist in der oben beschriebenen Art und Weise möglich.

Ferner sind Instrumente sowie Knochenplatten-Fixiervorrichtung der eingangs beschriebenen Art aus der EP 0 857 466 A1 und der US 6,379,363 bekannt.

Mit dem bekannten Instrument ist jedoch ein definierter Transport des Verbindungsglieds relativ zum zweiten Knochenanlageelement nicht eindeutig gewährleistet. Ferner ist es schwierig, ein glattes Verbindungsglied sicher zu greifen. Bei strukturierten Verbindungsgliedern ergibt sich das Problem, dass sich eine Struktur des Verbindungsglieds in die Spannbacken eingraben und zu einer Beschädigung derselben führen kann. In jedem Fall besteht die Gefahr, bei hohen auf das zweite Werkzeugelement wirkenden Zugkräften, dass die Spannbacken am Verbindungsglied abrutschen können. Ferner läßt sich das Instrument nur schwer reinigen, wenn die Spannbacken Beschädigungen aufgrund scharfkantiger Strukturen der Verbindungsglieder aufweisen.

Es ist daher Aufgabe der vorliegenden Erfindung, ein chirurgisches Instrument der eingangs beschriebenen Art so zu verbessern, dass die Reinigbarkeit des Instruments verbessert wird.

Diese Aufgabe wird bei einem chirurgischen Instrument der eingangs beschriebenen Art erfindungsgemäß dadurch gelöst, dass die Verzahnung des zweiten Werkzeugelements eine Teilung aufweist, welche einem ganzzahligenechten Vielfachen einer Teilung der Vorsprungverzahnung entspricht.

Das erfindungsgemäße Instrument ermöglicht es, den Vorsprung des Verbindungsglieds definiert und mit einer vorgegebenen Schrittweite durch das Instrument hindurchzureichen. Die Ausbildung von Aufnahmen zum Aufnehmen des Vorsprungs ermöglicht es, die Aufnahmen entsprechend groß auszubilden, so dass das zweite Werkzeugelement und damit das gesamte Instrument gut gereinigt werden können. In der Eingriffsposition ist eine Bewegung des Verbindungsglieds relativ zum zweiten Werkzeugelement in Längsrichtung nicht möglich. Dadurch kann das zweite Werkzeugelement nicht vom mittels des Vorsprungs in einer Aufnahme gehaltenen Verbindungsglied abrutschen. Damit ergibt sich eine besonders einfache Ausgestaltung des zweiten Werkzeugelements, weist das zweite Werkzeugelement eine eine Mehrzahl von Zähnen umfassende Verzahnung auf und umfasst die Verzahnung die Aufnahmen. Denkbar wäre es, den Vorsprung in Form eines Kopfes auszubilden. Um jedoch eine Verbindung zwischen dem zweiten Werkzeugelement und dem Vorsprung zu verbessern, weist der Vorsprung eine mindestens zwei Zähne umfassende Vorsprungverzahnung auf. Dadurch ist es möglich, dass wahlweise ein Zahn der Verzahnung des zweiten Werkzeugelements zwischen die mindestens zwei Zähne der Vorsprungverzahnung eingreift. Denkbar ist es auch, dass der Vorsprung als Ganzes, also auch seine mindestens zwei Zähne umfassende Vorsprungverzahnung, in eine einzelne Aufnahme der Verzahnung des zweiten Werkzeugelements einführbar ist. Das Reinigen des zweiten Werkzeugelements wirt erleichtert, dadurch, dass die Verzahnung des zweiten Werkzeugelements erfindungsgemäß eine Teilung aufweist, welche einem ganzzahligenechten Vielfachen einer Teilung der Vorsprungverzahnung entspricht. Dadurch ergeben sich besonders große Abstände der Zähne der Verzahnung des zweiten Werkzeugelements. Insbesondere kann ein Teilungsverhältnis 2:1 oder 3:1 betragen.

Günstig ist es, wenn das zweite Werkzeugelement in einer relativ zum ersten Werkzeugelement distalen Stellung mit dem Vorsprung in der Eingriffsposition in Eingriff bringbar ist, wenn das zweite Werkzeugelement in der Eingriffsposition in proximaler Richtung von der distalen Stellung in eine vom ersten Werkzeugelement entferntere proximale Stellung bringbar ist und wenn das zweite Werkzeugelement in der proximalen Stellung von der Eingriffsposition in eine Löseposition bringbar ist, in welcher das zweite Werkzeugelement und der Vorsprung außer Eingriff sind. Ein derart aufgebautes Instrument ermöglicht es, den Vorsprung mit dem zweiten Werkzeugelement zu fassen und in proximaler Richtung zu bewegen, so dass das am ersten Werkzeugelement anliegende zweite Knochenanlageelement relativ zum Vorsprung des Verbindungsglieds bewegt wird. Zum Nachfassen, also einem nochmaligen Greifen des Vorsprungs mit dem zweiten Werkzeugelement, läßt sich die Eingriffsposition lösen, das heißt, das zweite Werkzeugelement und der Vorsprung sind wieder relativ zueinander in Längsrichtung verschiebbar. Dies ist nur in der Löseposition möglich.

Vorteilhaft ist es, wenn das zweite Werkzeugelement in der Löseposition von der proximalen Stellung in die distale Stellung bringbar ist. Der Vorsprung behält dann seine relative Position zum zweiten Knochenanlageelement bei, während das zweite Werkzeugelement am Vorsprung vorbei in die distale Stellung gebracht werden kann. Auf diese Weise läßt sich ein schrittweiser Transport des Vorsprungs mit dem Instrument in proximaler Richtung realisieren.

Damit der Vorsprung sicher am zweiten Werkzeugelement in Längsrichtung unbeweglich haltbar ist, ist es vorteilhaft, wenn das zweite Werkzeugelement quer zur Längsrichtung relativ zum Vorsprung bewegbar ist. Es ermöglicht auf diese Weise quasi eine Verriegelung des Vorsprungs am zweiten Werkzeugelement.

Besonders einfach wird der Aufbau des Instruments, wenn das zweite Werkzeugelement einen ersten und einen zweiten Spannbacken umfasst und wenn mindestens einer der beiden Spannbacken die Aufnahmen trägt. Der Vorsprung kann auf diese Weise zwischen den beiden Spannbacken gehalten werden. Selbstverständlich ist es auch möglich, beide Spannbacken mit Aufnahmen zu versehen, so dass der Vorsprung beidseitig durch Aufnahmen der Spannbacken gehalten werden kann.

Um einen Transport des Vorsprungs vom zweiten Knochenanlageelement weg in definierter Weise durchzuführen, kann es vorteilhaft sein, wenn das Instrument derart ausgebildet ist, dass von einem Transportschritt zu einem nachfolgenden Transportschritt der Vorsprung mindestens um einen Transportweg in proximaler Richtung transportierbar ist und wenn der Transportweg dem kleineren der Zahnabstände der Verzahnung und der Vorsprungverzahnung entspricht. Dies ermöglicht es, einen definierten kleinsten Transportweg durch die Form der Verzahnung beziehungsweise der Vorsprungverzahnung vorzugeben. Selbstverständlich kann ein tatsächlicher Transportweg oder -hub einem ganzzähligen Vielfachen des kleinsten Transportwegs entsprechen.

Um eine Relativbewegung in Längsrichtung in der Eingriffsposition zwischen dem Vorsprung und dem zweiten Werkzeugelement zu vermeiden, kann es vorteilhaft sein, wenn der mindestens eine Zahn der Verzahnung quer zur Längsrichtung in die Halteaufnahme einführbar ist.

Um eine Beschädigung des zweiten Werkzeugelements und auch des Verbindungsglieds zu vermeiden, ist es vorteilhaft, wenn die Aufnahmen kantenfrei ausgebildet sind. Die kantenfreie Ausgestaltung hat darüber hinaus den Vorteil, dass der Vorsprung beim Eintauchen in eine Aufnahme durch vorteilhafte Rundungen der Aufnahmen in die Aufnahmen hinein geführt wird.

Für eine einfache Handhabung des Instruments ist es günstig, wenn das Instrument einen Grundkörper und mindestens ein am Grundkörper beweglich gelagertes Betätigungselement aufweist und wenn durch eine Bewegung des Betätigungselements relativ zum Grundkörper eine Zugkraft auf das zweite Werkzeugelement in Längsrichtung von dem ersten Werkzeugelement weg übertragbar ist. Dadurch läßt sich das Werkzeugelement auf einfache Weise in Längsrichtung bewegen.

Gemäß einer bevorzugten Ausführungsform der Erfindung kann vorgesehen sein, dass durch eine Bewegung des Betätigungselements relativ zum Grundkörper eine Haltekraft auf das zweite Werkzeugelement quer zur Längsrichtung übertragbar ist. Dies gestattet es, durch die Bewegung des Betätigungselements gleichzeitig eine Haltekraft und eine Zugkraft auf das zweite Werkzeugelement auszuüben. Eine Bedienperson muß somit nur das Betätigungselement bewegen und kann dadurch den Vorsprung vom ersten Werkzeugelement weg bewegen.

Vorteilhaft ist es, wenn ein Kraftumlenkglied zum Umlenken einer in Längsrichtung wirkenden Zugkraft in die quer zur Längsrichtung wirkende Haltekraft vorgesehen ist. Durch Ausüben einer Zugkraft wird nicht nur das zweite Werkzeugelement in Richtung der Zugkraft bewegt, sondern gleichzeitig kann mit dem zweiten Werkzeugelement eine Haltekraft auf das Verbindungsglied, insbesondere auf den Vorsprung, ausgeübt werden.

Ein besonders kompakter Aufbau des Instruments ergibt sich, wenn der mindestens eine Spannbacken an dem Kraftumlenkglied anliegt und an diesem während einer Bewegung des Kraftumlenkglieds in Längsrichtung führbar ist. Dies ist beispielsweise durch schräge Aufgleitflächen am Kraftumlenkglied zu realisieren. Ferner kann eine Kraft direkt vom Kraftumlenkglied auf den mindestens einen Spannbacken übertragen werden, weitere Teile werden hierfür nicht benötigt.

Damit von dem Kraftumlenkglied Zugkräfte übertragen werden können, ist es vorteilhaft, wenn auf das Kraftumlenkglied von dem mindestens einen Betätigungsglied eine Zugkraft übertragbar ist.

Um einen Rückstoß oder ein Rückschlagen des zweiten Werkzeugelements am Instrument zu vermeiden, kann vorgesehen sein, dass sich der mindestens eine Spannbacken in Längsrichtung federnd am Kraftumlenkglied abstützt. Dadurch wird er stets unter Vorspannung am Kraftumlenkglied gehalten, wodurch eine besonders schonende Anwendung des Instruments möglich wird.

Um zusätzlich Rückstoßkräfte aufzunehmen, falls das mindestens eine Betätigungselement schlagartig gelöst wird, kann sich das Kraftumlenkglied federnd am Grundkörper abstützen.

Eine Beschädigung des Instruments kann wirkungsvoll vermieden werden, wenn eine Zugkraftbegrenzungsvorrichtung zum Begrenzen der Zugkraft in Längsrichtung vorgesehen ist. Unabhängig davon, wie groß eine von einer Bedienperson ausgeübte Kraft auf das Betätigungselement ist, wird eine maximale Zugkraft mittels der Zugkraftbegrenzungsvorrichtung begrenzt.

Um Kräfte vom Betätigungselement begrenzt auf das Kraftumlenkglied zu übertragen, ist es günstig, wenn mit der Zugkraftbegrenzungsvorrichtung eine von dem mindestens einen Betätigungselement eingeleitete Kraft begrenzt auf das Kraftumlenkglied übertragbar ist.

Besonders gute Dämpfungseigenschaften lassen sich für das Instrument erreichen, wenn sich das Kraftumlenkglied federnd an der Zugkraftbegrenzungsvorrichtung abstützt.

Um die Dämpfungseigenschaften des Instruments weiter zu verbessern, kann sich die Zugkraftbegrenzungsvorrichtung federnd am Grundkörper abstützen. Durch die federnde Abstützung werden Rückstoßkräfte abgemildert, die bei einem schlagartigen Loslassen des mindestens einen Betätigungselements auftreten können.

Um eine Trennung einer Hub- und Zugbewegung des zweiten Werkzeugelements zu erreichen, ist es vorteilhaft, wenn der mindestens eine Spannbacken an einem in Längsrichtung am Grundkörper verschieblich gelagerten Schub- und Zugelement gelagert ist und wenn von dem mindestens einen Betätigungselement eine Zugkraft auf das Schub- und Zugelement übertragbar ist.

Die eingangs gestellte Aufgabe wird bei einer Knochenplatten-Fixiervorrichtung der eingangs beschriebenen Art erfindungsgemäß dadurch gelöst, dass die Verzahnung des zweiten Werkzeugelements eine Teilung aufweist, welche einem ganzzahligenechten Vielfachen einer Teilung der Vorsprungverzahnung entspricht.

Eine besonders sichere Verbindung in der Eingriffsposition ergibt sich, wenn der Vorsprung formschlüssig in die Aufnahmen einführbar ist. Wenn der Vorsprung korrespondierend zu einer Aufnahme ausgebildet ist, kann es auch nicht zu Beschädigungen des zweiten Werkzeugelements kommen. Außerdem läßt sich das zweite Werkzeugelement auf einfache Weise reinigen, wenn der Vorsprung und die Aufnahme entsprechend groß genug ausgebildet sind.

Vorteilhaft ist es, wenn der Vorsprung eine Halteaufnahme zum Aufnehmen mindestens eines Zahns der Verzahnung aufweist. Dies hat den Vorteil, dass einerseits der Vorsprung als Ganzes in eine Aufnahme des zweiten Werkzeugelements einführbar ist und dass andererseits ein Zahn der Verzahnung in die Halteaufnahme einführbar ist. Damit läßt sich eine doppelte Verbindung realisieren, beispielsweise in Form von zwei formschlüssig ineinandergreifenden Zähnen beziehungsweise Lücken zwischen zwei Zähnen.

Besonders einfach wird der Aufbau der Vorrichtung, wenn die Vorsprungverzahnung die Halteaufnahme umfasst.

Günstig ist es, wenn die Halteaufnahme eine Ringnut umfasst. Eine solche läßt sich an dem Vorsprung oder direkt an dem Verbindungsglied besonders einfach herstellen.

Damit durch den Vorsprung keine Beschädigungen am zweiten Werkzeugelement hervorgerufen werden können, ist es von Vorteil, wenn der Vorsprung kantenfrei ausgebildet ist. Auf diese Weise kann er noch besser in eine Aufnahme des zweiten Werkzeugelements hineingleiten. Hilfreich sind dabei abgerundete Formen des Vorsprungs.

Weitere vorteilhafte Weiterbildungen sind in den Unteransprüchen angegeben.

Die nachfolgende Beschreibung einer bevorzugten Ausführungsform der Erfindung dient im Zusammenhang mit der Zeichnung der näheren Erläuterung. Es zeigen:
- Figur 1:: eine erfindungsgemäße Spannzange mit Spannbacken in einer dis- talen Lösestellung;
- Figur 2:: das Instrument aus Figur 1 mit den Spannbacken in einer distalen Eingriffsposition;
- Figur 3:: das Instrument aus Figur 1 mit den Spannbacken in einer proxima- len Zugstellung;
- Figur 4:: das Instrument aus Figur 3 bei wirkender Zugkraftbegrenzung;
- Figur 5:: eine mögliche erste Eingriffsposition eines Vorsprungs eines Verbin- dungsglieds an Zähnen der Spannbacken; und
- Figur 6:: eine zweite mögliche Eingriffsposition des Vorsprungs an den Zäh- nen der Spannbacken.

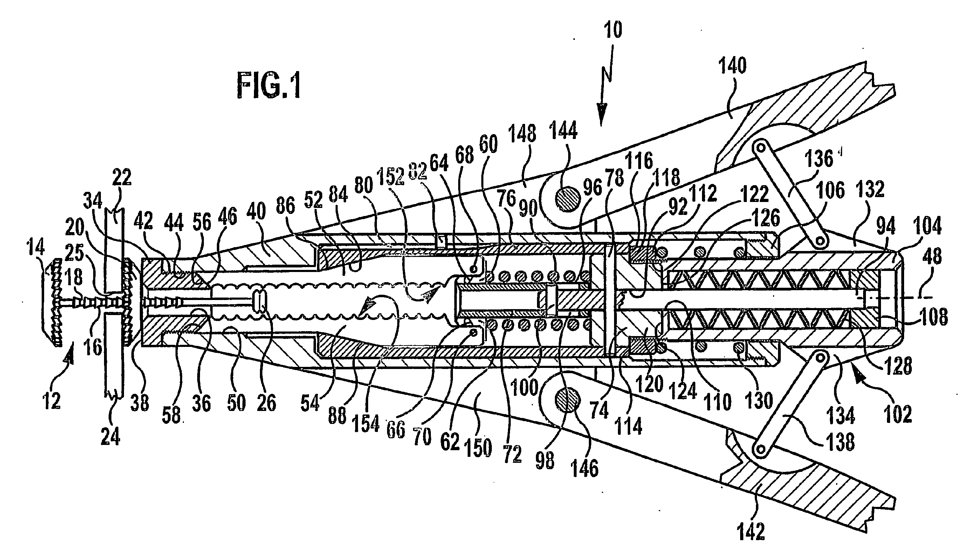

In den Figuren 1 bis 4 ist ein erfindungsgemäßes Instrument in Form einer chirurgischen Spannzange dargestellt. Die Spannzange 10 dient zum Anlegen eines nietartigen Fixierungselements 12, welches ein erstes Anlageelement 14 mit einem von diesem abstehenden, mit Rückhaltevorsprüngen 16 versehenen langgestreckten Schaft 18 sowie ein zweites Anlageelement 20 umfasst, welches relativ zum ersten Anlageelement 14 auf dem Schaft 18 in Richtung auf das erste Anlageelement 14 hin verschiebbar ist. Eine Verschiebung des zweiten Anlageelements 20 relativ zum ersten Anlageelement 14 von diesem weg ist aufgrund der in diese Richtung wirkenden Rückhaltevorsprünge 16 nicht möglich. Zwischen den Anlageelementen 14 und 20 können zwei getrennte Knochenteile 22 und 24, welche beispielsweise Teile eines menschlichen Schädelknochen bilden, miteinander fixiert werden, indem die beiden Anlageelemente 14 und 20 die Knochenteile 22 und 24 beidseitig zwischen sich einklemmen.

An einem vom ersten Anlageelement 14 weg weisenden Ende des Schafts 18 ist ein ringförmiger Vorsprung 26 angeordnet, welcher eine ringförmige Einschnürung 28 aufweist. Der Vorsprung 26 ist auf diese Weise quasi mit einer Verzahnung umfassend zwei Zähne 30 und 32 versehen.

Mittels der Spannzange 10 ist eine Relativbewegung zwischen den beiden Anlageelementen 14 und 20 realisierbar. Hierfür umfasst die Spannzange 10 ein erstes Werkzeugelement in Form einer mit einer Längsbohrung 36 versehenen Einschraubhülse 34, die eine ringförmige, in distaler Richtung weisende Anlagefläche 38 zum Anlegen am zweiten Anlageelement 20 aufweist. Die Längsbohrung 36 ist so bemessen, dass der Schaft 18 mit dem Vorsprung 26 durch die Einschraubhülse 34 hindurchgeführt werden kann.

Die Einschraubhülse 34 ist mit einem Außengewindeabschnitt 42 versehen, welcher zu einem Innengewindeabschnitt 44 an einem distalen Ende eines Grundkörpers 40 der Spannzange 10 korrespondiert. An ihrem proximalen Ende weist die Einschraubhülse 34 eine Kegelmantelfläche 46 auf, die in proximaler Richtung weist. Eine Spitze eines von der Kegelmantelfläche 46 definierten Kegels würde auf einer Längsachse 48 der Spannzange 10 liegen, welche gleichzeitig eine Symmetrieachse der Spannzange 10 und des Fixierungselementes 12 bildet.

Der Grundkörper 40 ist in Form einer langgestreckten Hülse ausgebildet und weist an die Kegelmantelfläche 46 angrenzend einen ringförmigen Anlageabschnitt 50 für zwei symmetrisch zur Längsachse 48 angeordnete langgestreckte Spannbacken 52 und 54 auf. Distalseitig sind die Spannbacken 52 und 54 jeweils mit einer zur Kegelmantelfläche 46 korrespondierenden schrägen Aufgleitfläche 56 beziehungsweise 58 versehen. Proximalseitig sind freie Enden der Spannbacken 52 und 54 an Lagerlappen 60 und 62 sowohl schwenkbar als auch verschiebbar gelagert, und zwar indem ein drehfest an den Spannbacken 52 beziehungsweise 54 quer zur Längsachse 48 orientierter Stift 68 beziehungsweise 70 einen an den Lagerlappen 60 beziehungsweise 62 schräg von der Längsachse 48 in proximaler Richtung weisenden Schlitz 64 beziehungsweise 66 durchsetzt. Die Lagerlappen 60 und 62 sind distalseitig radial abstehend an einer Zughülse 72 angeordnet, welche proximalseitig mit einem rotationssymmetrisch zur Längsachse 48 geformten Lagerzapfen 74 verbunden ist. Der Lagerzapfen 74 ist distalseitig in ein proximales Ende einer Klemmhülse 76 mittels eines quer zur Längsachse 48 sowohl den Lagerzapfen 74 als auch die Klemmhülse 76 durchsetzenden Bolzens 78 drehfest und auch axial verschieblich gesichert.

Die Klemmhülse 76 ist im Grundkörper 40 axial verschieblich gelagert und gegen ein Verdrehen relativ zum Grundkörper 40 gesichert durch eine sich außen von einem proximalen Ende der Klemmhülse 76 weg erstreckenden Längsnut 80, in welche ein innen von der Klemmhülse 76 in Richtung auf die Längsachse 48 hin weisend abstehender Sicherungszapfen 82 eintaucht. Die Klemmhülse 76 weist distalseitig einen sich verringernden Innendurchmesser auf, wodurch eine Umlenkfläche 84 gebildet wird, die schräg in proximaler Richtung auf die Längsachse 48 hin weist. Die Spannbacken 52 und 54 weisen zur Umlenkfläche 84 korrespondierend geneigte Gleitflächen 86 beziehungsweise 88 auf, die in einer in Figur 1 dargestellten Ausgangsstellung im wesentlichen vollständig an der Umlenkfläche 84 anliegen.

Eine die Zughülse 72 umgebende Spiralfeder 90 stützt sich einerseits an den Lagerlappen 60 und 62, andererseits am Lagerzapfen 74 ab. Die Spiralfeder 90 drückt somit die Spannbacken 52 und 54 in distaler Richtung mit ihren Gleitflächen 86 und 88 gegen die Umlenkfläche 84 sowie die Aufgleitflächen 56 und 58 gegen die Kegelmantelfläche 46.

Der Lagerzapfen 74 weist eine zentrale Bohrung 92 auf, in welcher ein zylindrischer langgestreckter Zugbolzen 94 eingesetzt und mittels des Bolzens 78 drehfest und axial unverschieblich am Lagerzapfen 74 gehalten ist. Distalseitig ist der Zugbolzen 94 in der Zughülse 72 verschiebbar gelagert, welche zwei sich parallel zur Längsachse 48 erstreckende Führungsschlitze 96 und 98 aufweist, in welche ein quer zur Längsachse 48 den Zugbolzen 94 durchsetzender Führungsstift 100 eintaucht und auf diese Weise die Zughülse 72 axial verschieblich und gegen eine Verdrehung gesichert am Zugbolzen 94 hält.

Proximalseitig ist der Zugbolzen 94 mit einer insgesamt mit dem Bezugszeichen 102 versehenen Zugkraftbegrenzung 102 verbunden. Diese umfasst eine Lagerhülse 104, welche längsverschieblich an einem in ein proximales Ende des Grundkörpers 40 eingeschraubten Lagerring 106 axial verschieblich geführt ist. Die Lagerhülse 104 führt in ihrem Inneren einen ringförmigen Kopf 108, welcher drehfest mit einem proximalen Ende des Zugbolzens 94 verbunden ist. Distalseitig wird der Zugbolzen 94 an einer zentralen axialen Hülsenbohrung 110 axial verschieblich geführt.

Auf ein distales Ende der Lagerhülse 104 ist außen ein Anschlagring 112 aufgeschraubt, welcher eine in distaler Richtung weisende Anschlagfläche 114 bildet. An der Anschlagfläche 114 liegt in der in Figur 1 dargestellten Grundstellung ein distales Ende 116 der Klemmhülse 76 sowie ein Ringvorsprung 118 des Lagerzapfens 74 an. Ein gegenüber dem Ringvorsprung 118 im Durchmesser verringerter Zapfenabschnitt 120 taucht in eine korrespondierende zylindrische Ausnehmung 122 der Lagerhülse 104 ein, welche in distaler Richtung offen ist. Ein proximales Ende 126 des Lagerzapfens 74 stößt an einem Boden 124 der Ausnehmung 122 an, welche von der Hülsenbohrung 110 durchsetzt ist.

Den Zugbolzen 94 umgebend ist in der Lagerhülse 104 ein Tellerfederblock 128 angeordnet, der sich einerseits am Boden 124 und andererseits am Kopf 108 abstützt und so den Lagerzapfen 74 unter Vorspannung in der Ausnehmung 122 hält. Die Lagerhülse an ihrem distalseitigen Ende umgebend ist eine weitere Spiralfeder 130 innerhalb des Grundkörpers 40 angeordnet, die sich einerseits am Anschlagring 112 und andererseits am Lagerring 106 abstützt. Sie drückt damit insgesamt die Lagerhülse 104 in distaler Richtung.

Proximalseitig sind an der Lagerhülse 104 radial abstehend symmetrisch zwei Lagerböcke 132 und 134 angeordnet, an denen jeweils ein stabförmiger Lenker 136 beziehungsweise 138 schwenkbar gelagert ist. Die Lenker 136 und 138 sind ferner mit jeweils einem Schwenkgriff 140 beziehungsweise 142 schwenkbar verbunden. Die Schwenkgriffe 140 und 142 sind mittels zweier quer zur Längsachse 48 orientierter Gelenkbolzen 144 beziehungsweise 146 an radial von dem Grundkörper 40 abstehenden Lagerlappen 148 und 150 schwenkbar gehalten.

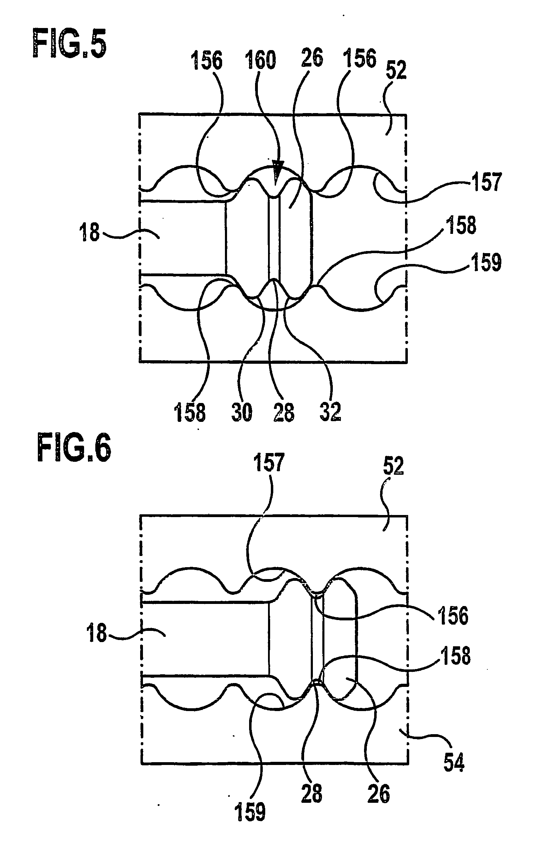

Die Spannbacken 52 und 54 sind jeweils mit einer Verzahnung 152 beziehungsweise 154 versehen, welche jeweils eine Vielzahl von in Richtung auf die Längsachse 48 hin weisenden Zähnen 156 und 158 aufweisen. Jeweils zwischen zwei Zähnen 156 und 158 sind Aufnahmen 157 beziehungsweise 159 bildende Vertiefungen ausgebildet. Die Zähne 156 und 158 sind allesamt verrundet. Ein Abstand der Zähne 156 und 158 voneinander ist so gewählt, dass der Vorsprung 26 als Ganzes zwischen zwei Zähne 156 und 158 einführbar ist. Eine solche Eingriffsposition ist in Figur 5 dargestellt.

Die Form einer Spitze der Zähne 156 und 158 entspricht jedoch auch im wesentlichen der Form der Einschnürung 28 des Vorsprungs 26 am Schaft 18, so dass jeweils ein Zahn 156 und 158 der Spannbacken 52 und 54 in die Einschnürung 28 eintauchen kann. Eine solche Eingriffsposition ist in Figur 6 dargestellt. Die Verzahnungen 152 und 154 sind so gewählt, dass die Zähne 30 und 32 des Vorsprungs 26 halb so weit voneinander entfernt sind wie jeweils zwei Zähne 156 beziehungsweise 158. Dadurch entspricht eine Teilung der Verzahnungen 152 und 154 zweimal der Teilung einer Verzahnung 160 des Vorsprungs 26. Damit lassen sich Eingriffspositionen definieren, die dem halben Abstand der Teilung der Verzahnungen 152 und 154 entsprechen. Zwei derartige, in einem solchen Abstand voneinander getrennte Eingriffspositionen sind in den Figuren 5 und 6 dargestellt.

In Verbindung mit den Figuren 1 bis 4 wird nachfolgend näher erläutert, wie mittels der Spannzange 10 das zweite Anlageelement 20 relativ zum Schaft 18 in Richtung auf das erste Anlageelement 14 hin verschoben werden kann.

Zunächst werden die beiden Anlageelemente 14 und 20 beidseitig der zwei miteinander zu verbindenden Knochenteile 22 und 24 an diese angelegt und der Schaft 18 durch einen Knochenspalt 25 hindurchgeführt. Der Schaft 18 mit dem Vorsprung 26 wird durch die Einschraubhülse 34 hindurchgesteckt. Die Einschraubhülse 34 wird an das zweite Anlageelement 20 angelegt. Diese Grundstellung ist in Figur 1 dargestellt.

Durch Verschwenken der Schwenkgriffe 140 und 142 in Richtung auf die Längsachse 48 hin, wird die Lagerhülse 104 in proximaler Richtung gezogen und drückt die Spiralfeder 130 zusammen. Solange die von den Schwenkgriffen 140 und 142 ausgeübte Kraft kleiner als die von dem Tellerfederblock 128 ausgeübte Kraft ist, wird der Lagerzapfen 74 in der Ausnehmung 122 der Lagerhülse 104 gehalten. Zusammen mit dem Lagerzapfen 74 wird die Klemmhülse 76 in proximaler Richtung gezogen, wodurch die Gleitflächen 86 und 88 der Spannbacken 52 und 54 an der Umlenkfläche 84 der Klemmhülse 76 aufgleiten. Die Umlenkfläche 84 wirkt somit als Umlenkglied, mit welchem eine in Richtung der Längsachse 48 wirkende Zugkraft in eine Schubkraft in Richtung auf die Längsachse 48 hin umgelenkt wird. Die Spannbacken 52 und 54 werden zwangsgeführt in Richtung auf die Längsachse 48 hin bewegt, wobei eine Führung einerseits durch die an der Kegelmantelfläche 46 anliegenden Aufgleitflächen 56 und 58 realisiert wird, andererseits mittels der in den Schlitzen 64 und 66 geführten Stifte 68 und 70.

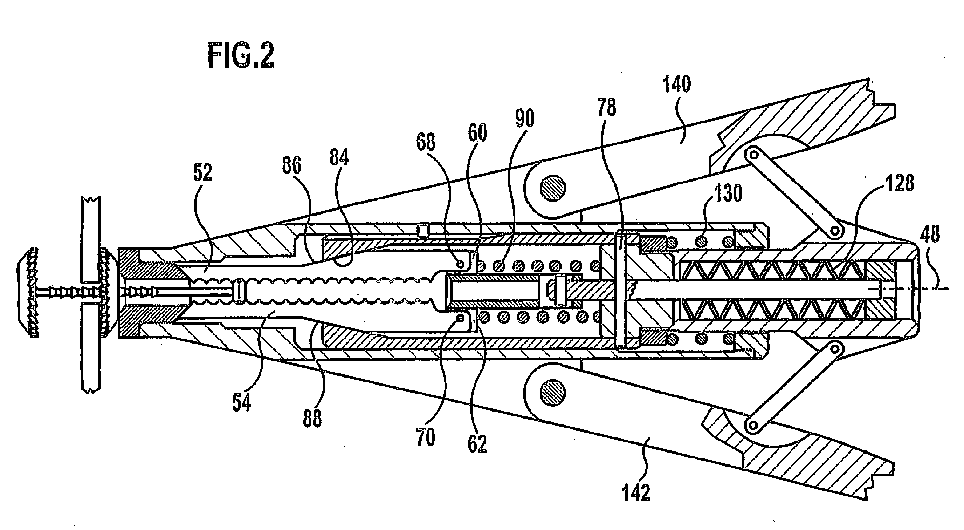

Die Spannbacken 52 und 54 können so weit in Richtung auf die Längsachse 48 hin bewegt werden, bis die Verzahnungen 152 und 154 mit dem Vorsprung 26 in Eingriff kommen. Hierzu gibt es zwei Eingriffspositionen, die im Zusammenhang mit den Figuren 5 und 6 bereits näher erläutert wurden. Figur 2 zeigt die Eingriffsposition der Spannbacken 52 und 54 am Vorsprung 26 in einer distalen Stellung derselben. Figur 5 entspricht einem vergrößerten Ausschnitt der Figur 2.

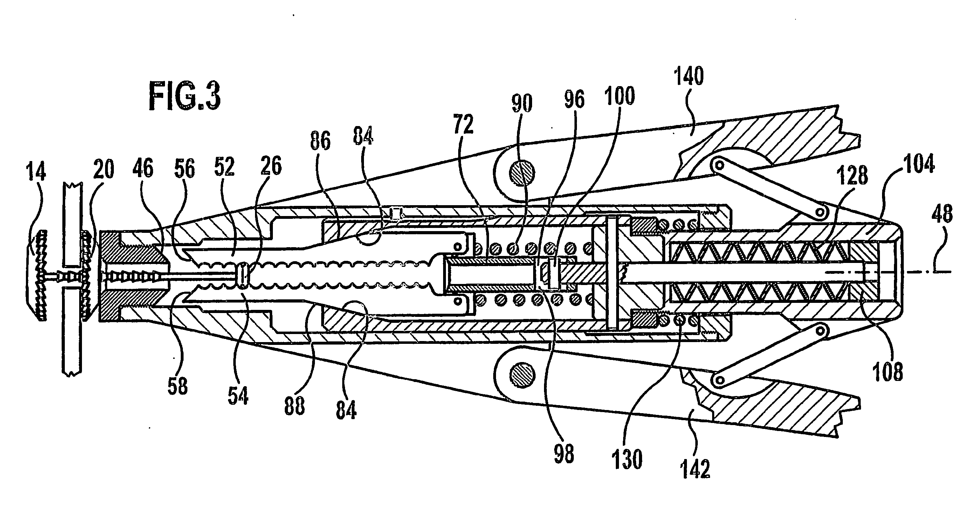

Werden die Schwenkgriffe 140 und 142 weiter in Richtung auf die Längsachse 48 hin verschwenkt, werden die Spannbacken 52 und 54 in proximaler Richtung mitgenommen. Die Kraft der Spiralfeder 90 reicht nicht aus, um die Spannbacken 52 und 54 weiter in distaler Richtung vorzuspannen. In Figur 3 ist eine Stellung der Spannzange 10 gezeigt, bei der der Vorsprung 26 relativ vom zweiten Anlageelement 20 weg bewegt wurde, so dass das zweite Anlageelement 20 bereits eine in Richtung auf das erste Anlageelement 14 hin veränderte Position einnimmt.

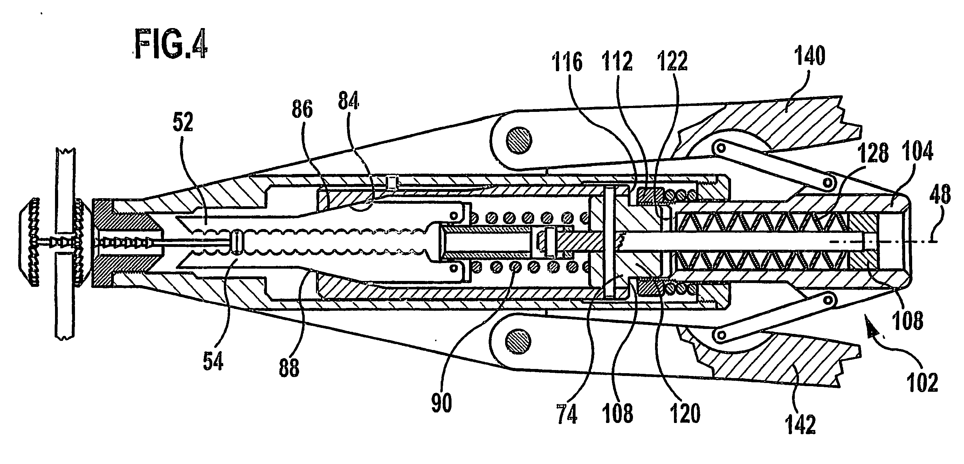

Werden die Schwenkgriffe 140 und 142 noch weiter in Richtung auf die Längsachse 48 hin verschwenkt, so beginnt die Zugkraftbegrenzung 102 zu wirken. Die auf die Lagerhülse 104 ausgeübte Zugkraft übersteigt nunmehr die vom Tellerfederblock 128 ausgeübte Kraft, wodurch der Tellerfederblock 128 komprimiert wird. Eine axiale Position der Klemmhülse 76 relativ zum Grundkörper 40 bleibt dadurch praktisch konstant. Dagegen wird die Spiralfeder 130 ebenso wie der Tellerfederblock 128 weiter zusammengedrückt. Diese Stellung ist in Figur 4 dargestellt.

Zum Nachfassen des Vorsprungs 26 mit den Spannbacken 52 und 54 werden die Schwenkgriffe 140 und 142 wieder von der Längsachse 48 weg verschwenkt. Dies kann beispielsweise mittels einer nicht dargestellten Blattfeder automatisch erfolgen. Durch entsprechende Auswahl der Spiralfedern 90 und 130 ermöglicht es die Anordnung der Spannzange 10, dass in der in Figur 3 dargestellten Zugposition beim Rückverschwenken der Schwenkgriffe 140 und 142 von der Längsachse 48 weg zunächst die Spannbacken 52 und 54 radial von der Längsachse 48 und vom Vorsprung 26 weg bewegt werden, wenn der Zug auf die Lagerhülse 104 reduziert wird. Damit geben die Spannbacken 52 und 54 den Vorsprung 26 am Schaft 18 frei. Ein weiteres Verschwenken der Schwenkgriffe 140 und 142 zurück in die in Figur 1 dargestellte Grundstellung führt dazu, dass die Spannbacken 52 und 54 in distaler Richtung bewegt werden, dabei jedoch nicht in Eingriff mit dem Vorsprung 26 stehen. Sobald die Aufgleitflächen 56 und 58 wieder an der Kegelmantelfläche 46 anliegen, kann der Vorsprung 26 in einem weiteren Transportschritt weiter in proximaler Richtung bewegt werden.

Insgesamt werden so viele Transportschritte in der oben beschriebenen Weise durchgeführt, bis die beiden Knochenteile 22 und 24 klemmend zwischen den beiden Anlageelementen 14 und 20 gehalten werden.

## Patentansprüche

1. Chirurgisches Instrument (10) zum Anlegen einer Knochenplatten-Fixiervorrichtung (12), welche ein erstes Knochenanlageelement (14) mit einem von diesem abstehenden stabförmigen, eine Längsrichtung (48) definierenden Verbindungsglied (18) und ein auf dem Verbindungsglied (18) in Richtung auf das erste Knochenanlageelement (14) verschiebbares zweites Knochenanlageelement (20) aufweist, mit einem an dem zweiten Knochenanlageelement (20) in einer Anlagestellung anlegbaren ersten Werkzeugelement (34) und einem von dem ersten Werkzeugelement (34) entfernbaren zweiten Werkzeugelement (52, 54), mit einer Transportvorrichtung zum schrittweisen Transportieren des Verbindungsglieds (18) mit dem zweiten Werkzeugelement (52, 54) in mehreren Transportschritten in einer proximalen Richtung weg von dem in der Anlagestellung am zweiten Knochenanlageelement (20) anliegenden ersten Werkzeugelement (34),
wobei das zweite Werkzeugelement (52, 54) mehrere Aufnahmen (157, 159) für einen am Verbindungsglied (18) abstehenden Vorsprung (26) aufweist,
wobei das zweite Werkzeugelement (52, 54) eine eine Mehrzahl von Zähnen (156, 158) umfassende Verzahnung (152, 154) aufweist und die Verzahnung (152, 154) die Aufnahmen (157, 159) umfasst,
wobei der Vorsprung (26) eine mindestens zwei Zähne (30, 32) umfassende Vorsprungverzahnung (160) aufweist
und wobei der Vorsprung bei jedem Transportschritt mindestens teilweise mit einer Aufnahme (157, 159) in einer Eingriffsposition in Eingriff bringbar und in dieser in Längsrichtung (48) unbeweglich am zweiten Werkzeugelement (52, 54) gehalten ist und wobei von einem Transportschritt zu einem nachfolgenden Transportschritt der Vorsprung (26) mit einer in proximalerer Richtung am zweiten Werkzeugelement (52, 54) angeordneten Aufnahme (157, 159) in Eingriff bringbar ist,
**dadurch gekennzeichnet, dass** die Verzahnung (152, 154) des zweiten Werkzeugelements (52, 54) eine Teilung aufweist, welche einem ganzzahligenechten Vielfachen einer Teilung der Vorsprungverzahnung (160) entspricht.

2. Instrument nach Anspruch 1, **dadurch gekennzeichnet, dass** das zweite Werkzeugelement (52, 54) in einer relativ zum ersten Werkzeugelement (34) distalen Stellung mit dem Vorsprung (26) in der Eingriffsposition in Eingriff bringbar ist, dass das zweite Werkzeugelement (52, 54) in der Eingriffsposition in proximaler Richtung von der distalen Stellung in eine vom ersten Werkzeugelement (34) entferntere proximale Stellung bringbar ist und dass das zweite Werkzeugelement (52, 54) in der proximalen Stellung von der Eingriffsposition in eine Löseposition bringbar ist, in welcher das zweite Werkzeugelement (52, 54) und der Vorsprung (26) außer Eingriff sind.

3. Instrument nach Anspruch 2, **dadurch gekennzeichnet, dass** das zweite Werkzeugelement (52, 54) in der Löseposition von der proximalen Stellung in die distale Stellung bringbar ist.

4. Instrument nach einem der voranstehenden Ansprüche, **dadurch gekennzeichnet, dass** das zweite Werkzeugelement (52, 54) quer zur Längsrichtung (48) relativ zum Vorsprung (26) bewegbar ist.

5. Instrument nach einem der voranstehenden Ansprüche, **dadurch gekennzeichnet, dass** das zweite Werkzeugelement einen ersten und einen zweiten Spannbacken (52, 54) umfasst und dass mindestens einer der beiden Spannbacken (52, 54) die Aufnahmen (157, 159) trägt.

6. Instrument nach einem der voranstehenden Ansprüche, **dadurch gekennzeichnet, dass** das Instrument derart ausgebildet ist, dass von einem Transportschritt zu einem nachfolgenden Transportschritt der Vorsprung (26) mindestens um einen Transportweg in proximaler Richtung transportierbar ist und dass der Transportweg dem kleineren der Zahnabstände der Verzahnung (152, 154) und der Vorsprungverzahnung (160) entspricht.

7. Instrument nach einem der voranstehenden Ansprüche, **dadurch gekennzeichnet, dass** die Mehrzahl von Zähnen (156, 158) der Verzahnung (152, 154) quer zur Längsrichtung (48) in eine Halteaufnahme (28) einführbar ist, und dass der Vorsprung (26) die Halteaufnahme (28) zum Aufnehmen mindestens eines Zahns (156, 158) der Verzahnung (152, 154) aufweist.

8. Instrument nach einem der voranstehenden Ansprüche, **dadurch gekennzeichnet, dass** die Aufnahmen (157, 159) kantenfrei ausgebildet sind.

9. Instrument nach einem der voranstehenden Ansprüche, **dadurch gekennzeichnet, dass** das Instrument (10) einen Grundkörper (40) und mindestens ein am Grundkörper (40) beweglich gelagertes Betätigungselement (140, 142) aufweist und dass durch eine Bewegung des Betätigungselements (140, 142) relativ zum Grundkörper (40) eine Zugkraft auf das zweite Werkzeugelement (52, 54) in Längsrichtung (48) von dem ersten Werkzeugelement (34) weg übertragbar ist.

10. Instrument nach Anspruch 9, **dadurch gekennzeichnet, dass** durch eine
Bewegung des Betätigungselements (140, 142) relativ zum Grundkörper (40) eine Haltekraft auf das zweite Werkzeugelement (52, 54) quer zur Längsrichtung (48) übertragbar ist.

11. Instrument nach Anspruch 10, **dadurch gekennzeichnet, dass** ein Kraftumlenkglied (84) zum Umlenken einer in Längsrichtung (48) wirkenden Zugkraft in die quer zur Längsrichtung (48) wirkende Haltekraft vorgesehen ist.

12. Instrument nach Anspruch 11, **dadurch gekennzeichnet, dass** der mindestens eine Spannbacken (52, 54) an dem Kraftumlenkglied (84) anliegt und an diesem während einer Bewegung des Kraftumlenkglieds (84) in Längsrichtung (48) führbar ist.

13. Instrument nach einem der Ansprüche 11 oder 12, **dadurch gekennzeichnet, dass** auf das Kraftumlenkglied (84) von dem mindestens einen Betätigungsglied (140, 142) eine Zugkraft übertragbar ist.

14. Instrument nach einem der Ansprüche 11 bis 13, **dadurch gekennzeichnet, dass** sich der mindestens eine Spannbacken (52, 54) in Längsrichtung (48) federnd am Kraftumlenkglied (84) abstützt.

15. Instrument nach einem der Ansprüche 11 bis 14, **dadurch gekennzeichnet, dass** sich das Kraftumlenkglied (84) federnd am Grundkörper (40) abstützt.

16. Instrument nach einem der Ansprüche 10 oder 15, **dadurch gekennzeichnet, dass** eine Zugkraftbegrenzungsvorrichtung (102) zum Begrenzen der Zugkraft in Längsrichtung (48) vorgesehen ist.

17. Instrument nach Anspruch 16, **dadurch gekennzeichnet, dass** mit der Zugkraftbegrenzungsvorrichtung (102) eine von dem mindestens einen Betätigungselement (140, 142) eingeleitete Kraft begrenzt auf das Kraftumlenkglied (84) übertragbar ist.

18. Instrument nach einem der Ansprüche 16 oder 17, **dadurch gekennzeichnet, dass** sich das Kraftumlenkglied (84) federnd an der Zugkraftbegrenzungsvorrichtung (102) abstützt.

19. Instrument nach einem der Ansprüche 16 bis 18, **dadurch gekennzeichnet, dass** sich die Zugkraftbegrenzungsvorrichtung (102) federnd am Grundkörper (40) abstützt.

20. Instrument nach einem der Ansprüche 10 bis 19, **dadurch gekennzeichnet, dass** der mindestens eine Spannbacken (52, 54) an einem in Längsrichtung (48) am Grundkörper (40) verschieblich gelagerten Schub- und Zugelement (72) gelagert ist und dass von dem mindestens einen Betätigungselement (140, 142) eine Zugkraft auf das Schub- und Zugelement (72) übertragbar ist.

21. Knochenplatten-Fixiervorrichtung (12), welche ein erstes Knochenanlageelement (14) mit einem von diesem abstehenden stabförmigen, eine Längsrichtung (48) definierenden Verbindungsglied (18) und ein auf dem Verbindungsglied (18) in Richtung auf das erste Knochenanlageelement (14) verschiebbares zweites Knochenanlageelement (20) aufweist, wobei das Verbindungsglied (18) mit Rückhaltevorsprüngen (16) versehen ist, wodurch eine Verschiebung des zweiten Anlageelements (20) relativ zum ersten Anlageelement (14) von diesem weg aufgrund der in diese Richtung wirkenden Rückhaltevorsprünge (16) unmöglich ist, wobei das Verbindungsglied (18) einen abstehenden Vorsprung (26) aufweist, welcher eine mindestens zwei Zähne (30, 32) umfassende Vorsprungverzahnung (160) aufweist und wobei die Knochenplatten-Fixiervorrichtung (12) mit einem chirurgischen Instrument (10) anlegbar ist, welches Instrument ein an dem zweiten Knochenanlageelement (20) in einer Anlagestellung anlegbares erstes Werkzeugelement (34) und ein von dem ersten Werkzeugelement (34) entfernbares zweites Werkzeugelement (52, 54), eine Transportvorrichtung zum schrittweisen Transportieren des Verbindungsglieds (18) mit dem zweiten Werkzeugelement (52, 54) in mehreren Transportschritten in einer proximalen Richtung weg von dem in der Anlagestellung am zweiten Knochenanlageelement (20) anliegenden ersten Werkzeugelement (34) umfasst, wobei das zweite Werkzeugelement (52, 54) mehrere Aufnahmen (157, 159) für den Vorsprung (26) aufweist, wobei das zweite Werkzeugelement (52, 54) eine eine Mehrzahl von Zähnen (156, 158) umfassende Verzahnung (152, 154) aufweist und die Verzahnung (152, 154) die Aufnahmen (157, 159) umfasst, **dadurch gekennzeichnet, dass** die Verzahnung (152, 154) des zweiten Werkzeugelements (52, 54) eine Teilung aufweist, welche einem ganzzahligenechten Vielfachen einer Teilung der Vorsprungverzahnung (160) entspricht.

22. Knochenplatten-Fixiervorrichtung nach Anspruch 21, **dadurch gekennzeichnet, dass** der Vorsprung (26) formschlüssig in die Aufnahmen (157, 159) einführbar ist.

23. Knochenplatten-Fixiervorrichtung nach Anspruch 21 oder 22, **dadurch gekennzeichnet, dass** der Vorsprung (26) eine Halteaufnahme (28) zum Aufnehmen mindestens eines Zahns (156, 158) der Verzahnung (152, 154) aufweist.

24. Knochenplatten-Fixiervorrichtung nach Anspruch 23, **dadurch gekennzeichnet, dass** die Vorsprungverzahnung (160) die Halteaufnahme (28) umfasst.

25. Knochenplatten-Fixiervorrichtung nach Anspruch 23 oder 24, **dadurch gekennzeichnet, dass** die Halteaufnahme eine Ringnut (28) umfasst.

26. Knochenplatten-Fixiervorrichtung nach einem der Ansprüche 23 bis 25, **dadurch gekennzeichnet, dass** der Vorsprung (26) kantenfrei ausgebildet ist.

27. Knochenplatten-Fixiervorrichtung nach einem der Ansprüche 23 bis 26, **dadurch gekennzeichnet, dass** der Vorsprung (26) an einem vom ersten Knochenanlageelement (14) weg weisenden Ende des Verbindungsglieds (18) angeordnet ist.

28. Knochenplatten-Fixiervorrichtung nach einem der Ansprüche 23 oder 27, **dadurch gekennzeichnet, dass** der Vorsprung (26) ringförmig ausgebildet ist.

29. Knochenplatten-Fixiervorrichtung nach einem der Ansprüche 23 bis 28, **dadurch gekennzeichnet, dass** der Vorsprung (26) eine ringförmige Einschnürung (28) aufweist.

## Claims

1. Surgical instrument (10) for applying a bone plate fixing device (12) comprising a first bone contacting element (14) with a rod-shaped connecting member (18) projecting from the first bone contacting element and defining a longitudinal direction (48), and a second bone contacting element (20) displaceable on the connecting member (18) in a direction towards the first bone contacting element (14), with a first tool element (34) positionable in a contacting position on the second bone contacting element (20), and a second tool element (52, 54) removable from the first tool element (34), with a transportation device for stepwise transportation of the connecting member (18) with the second tool element (52, 54) in several transportation steps in a proximal direction away from the first tool element (34) resting in the contacting position on the second bone contacting element (20), the second tool element (52, 54) having several receptacles (157, 159) for a projection (26) protruding from the connecting member (18), the second tool element (52, 54) comprising a toothing (152, 154) having a plurality of teeth (156, 158), and the toothing (152, 154) comprising the receptacles (157, 159), the projection (26) having a projection toothing (160) comprising at least two teeth (30, 32), and with each transportation step the projection being at least partly engageable with a receptacle (157, 159) in an engagement position and being held therein immovably in longitudinal direction (48) on the second tool element (52, 54), and from one transportation step to a following transportation step the projection (26) being engageable with a receptacle (157, 159) arranged in a more proximal direction on the second tool element (52, 54), **characterized in that** the toothing (152, 154) of the second tool element (52, 54) has a pitch which corresponds to an integral real multiple of a pitch of the projection toothing (160).

2. Instrument in accordance with claim 1, **characterized in that** the second tool element (52, 54) is engageable in a distal position relative to the first tool element (34) with the projection (26) in the engagement position, **in that** the second tool element (52, 54) is movable in the engagement position in proximal direction from the distal position into a proximal position more removed from the first tool element (34), and **in that** the second tool element (52, 54) is transferable in the proximal position from the engagement position into a release position in which the second tool element (52, 54) and the projection (26) are disengaged.

3. Instrument in accordance with claim 2, **characterized in that** the second tool element (52, 54) is movable in the release position from the proximal position to the distal position.

4. Instrument in accordance with any one of the preceding claims, **characterized in that** the second tool element (52, 54) is movable transversely to the longitudinal direction (48) relative to the projection (26).

5. Instrument in accordance with any one of the preceding claims, **characterized in that** the second tool element comprises a first and a second clamping jaw (52, 54), and **in that** at least one of the two clamping jaws (52, 54) carries the receptacles (157, 159).

6. Instrument in accordance with any one of the preceding claims, **characterized in that** the instrument is so constructed that from one transportation step to a following transportation step the projection (26) is transportable over at least one transportation path in proximal direction, and **in that** the transportation path corresponds to the smaller of the tooth spacings of the toothing (152, 154) and the projection toothing (160).

7. Instrument in accordance with any one of the preceding claims, **characterized in that** the plurality of teeth (156, 158) of the toothing (152, 154) are introducible transversely to the longitudinal direction (48) into a holding receptacle (28), and **in that** the projection (26) comprises the holding receptacle (28) for receiving at least one tooth (156, 158) of the toothing (152, 154).

8. Instrument in accordance with any one of the preceding claims, **characterized in that** the receptacles (157, 159) are of edge-free design.

9. Instrument in accordance with any one of the preceding claims, **characterized in that** the instrument (10) comprises a main body (40) and at least one actuating element (140, 142) movably mounted on the main body (40), and **in that** by a movement of the actuating element (140, 142) relative to the main body (40) a pulling force is transmittable to the second tool element (52, 54) in longitudinal direction (48) away from the first tool element (34).

10. Instrument in accordance with claim 9, **characterized in that** by a movement of the actuating element (140, 142) relative to the main body (40) a holding force is transmittable to the second tool element (52, 54) transversely to the longitudinal direction (48).

11. Instrument in accordance with claim 10, **characterized in that** a force deflecting element (84) is provided for deflecting a pulling force acting in longitudinal direction (48) into the holding force acting transversely to the longitudinal direction (48).

12. Instrument in accordance with claim 11, **characterized in that** the at least one clamping jaw (52, 54) rests against the force deflecting element (84) and is guidable thereon during a movement of the force deflecting element (84) in longitudinal direction (48).

13. Instrument in accordance with claim 11 or 12, **characterized in that** a pulling force is transmittable from the at least one actuating element (140, 142) to the force deflecting element (84).

14. Instrument in accordance with any one of claims 11 to 13, **characterized in that** the at least one clamping jaw (52, 54) is resiliently supported on the force deflecting element (84) in longitudinal direction (48).

15. Instrument in accordance with any one of claims 11 to 14, **characterized in that** the force deflecting element (84) is resiliently supported on the main body (40).

16. Instrument in accordance with claim 10 or 15, **characterized in that** a pulling force limiter (102) is provided for limiting the pulling force in longitudinal direction (48).

17. Instrument in accordance with claim 16, **characterized in that** a force initiated by the at least one actuating element (140, 142) is transmittable to a limited extent to the force deflecting element (84) by the pulling force limiter (102).

18. Instrument in accordance with claim 16 or 17, **characterized in that** the force deflecting element (84) is resiliently supported on the pulling force limiter (102).

19. Instrument in accordance with any one of claims 16 to 18, **characterized in that** the pulling force limiter (102) is resiliently supported on the main body (40).

20. Instrument in accordance with any one of claims 10 to 19, **characterized in that** the at least one clamping jaw (52, 54) is mounted on a push-and-pull element (72) which is mounted on the main body (40) for displacement in longitudinal direction (48), and **in that** a pulling force is transmittable to the push-and-pull element (72) by the at least one actuating element (140, 142).

21. Bone plate fixing device (12), comprising a first bone contacting element (14) with a rod-shaped connecting member (18) projecting from the first bone contacting element and defining a longitudinal direction (48), and a second bone contacting element (20) displaceable on the connecting member (18) in a direction towards the first bone contacting element (14), the connecting member (18) being provided with retaining projections (16), whereby a displacement of the second contacting element (20) relative to the first contacting element (14) away from the first contacting element is not possible owing to the retaining projections (16) acting in this direction, the connecting member (18) comprising a protruding projection (26) having a projection toothing (160) comprising at least two teeth (30, 32), and the bone plate fixing device (12) being adapted to be applied with a surgical instrument (10), which instrument comprises a first tool element (34) positionable in a contacting position on the second bone contacting element (20), and a second tool element (52, 54) removable from the first tool element (34), a transportation device for stepwise transportation of the connecting member (18) with the second tool element (52, 54) in several transportation steps in a proximal direction away from the first tool element (34) resting in the contacting position on the second bone contacting element (20), the second tool element (52, 54) having several receptacles (157, 159) for the projection (26), the second tool element (52, 54) comprising a toothing (152, 154) having a plurality of teeth (156, 158), and the toothing (152, 154) comprising the receptacles (157, 159), **characterized in that** the toothing (152, 154) of the second tool element (52, 54) has a pitch which corresponds to an integral real multiple of a pitch of the projection toothing (160).

22. Bone plate fixing device in accordance with claim 21, **characterized in that** the projection (26) is introducible with a positive fit into the receptacles (157, 159).

23. Bone plate fixing device in accordance with claim 21 or 22, **characterized in that** the projection (26) has a holding receptacle (28) for receiving at least one tooth (156, 158) of the toothing (152, 154).

24. Bone plate fixing device in accordance with claim 23, **characterized in that** the projection toothing (160) comprises the holding receptacle (28).

25. Bone plate fixing device in accordance with claim 23 or 24, **characterized in that** the holding receptacle comprises a ring groove (28).

26. Bone plate fixing device in accordance with any one of claims 23 to 25, **characterized in that** the projection (26) is of edge-free design.

27. Bone plate fixing device in accordance with any one of claims 23 to 26, **characterized in that** the projection (26) is arranged at an end of the connecting member (18) pointing away from the first bone contacting element (14).

28. Bone plate fixing device in accordance with claim 23 or 27, **characterized in that** the projection (26) is of ring-shaped design.

29. Bone plate fixing device in accordance with any one of claims 23 to 28, **characterized in that** the projection (26) has a ring-shaped constriction (28).

## Revendications

1. Instrument chirurgical (10) pour l'application d'un dispositif de fixation de plaque d'ostéosynthèse (12), qui comprend un premier élément d'appui sur l'os (14) dont fait saillie un organe de liaison (18) en forme de tige définissant une direction longitudinale (48), et un deuxième élément d'appui sur l'os (20) pouvant coulisser sur l'organe de liaison (18) en direction du premier élément d'appui sur l'os (14), l'instrument comprenant un premier élément d'outil (34) qui, dans une position d'application, peut être appliqué contre le deuxième élément d'appui sur l'os (20), et un deuxième élément d'outil (52, 54) qui peut être éloigné du premier élément d'outil (34), l'instrument comprenant également un dispositif de transport pour transporter pas à pas l'organe de liaison (18), à l'aide du deuxième élément d'outil (52, 54), en plusieurs pas de transport, dans une direction proximale en s'éloignant du premier élément d'outil (34) qui, dans la position d'application, est appliqué contre le deuxième élément d'appui sur l'os (20), instrument
dans lequel le deuxième élément d'outil (52, 54) comporte plusieurs logements de réception (157, 159) pour une protubérance (26) en saillie de l'organe de liaison (18),
dans lequel le deuxième élément d'outil (52, 54) présente une denture (152, 154) comprenant une pluralité de dents (156, 158), et la denture (152, 154) englobant les logements de réception (157, 159),
dans lequel la protubérance (26) présente une denture de protubérance (160) englobant au moins deux dents (30, 32), et
dans lequel la protubérance, à chaque pas de transport, peut être amenée au moins partiellement en prise avec un logement de réception (157, 159), dans une position de prise, dans laquelle elle est alors maintenue de manière non mobile dans la direction longitudinale (48) sur le deuxième élément d'outil (52, 54), et
dans lequel la protubérance (26) peut, d'un pas de transport au pas de transport suivant, être amenée en prise avec un logement de réception (157, 159) agencé sur le deuxième élément d'outil (52, 54) en direction proximale,
**caractérisé en ce que** la denture (152, 154) du deuxième élément d'outil (52, 54) présente un pas qui correspond à un multiple entier naturel d'un pas de la denture de protubérance (160).

2. Instrument selon la revendication 1, **caractérisé en ce que** le deuxième élément d'outil (52, 54) peut, dans une position distale par rapport au premier élément d'outil (34), être amené en prise avec la protubérance (26) dans la position de prise, **en ce que** le deuxième élément d'outil (52, 54), dans la position de prise, peut être amené, en direction proximale, de la position distale dans une position proximale plus éloignée du premier élément d'outil (34), et **en ce que** le deuxième élément d'outil (52, 54) peut, dans la position proximale, être amené de la position de prise à une position de dégagement dans laquelle le deuxième élément d'outil (52, 54) et la protubérance (26) sont hors de prise.

3. Instrument selon la revendication 2, **caractérisé en ce que** le deuxième élément d'outil (52, 54) peut, dans la position de dégagement, être amené de la position proximale à la position distale.

4. Instrument selon l'une des revendications précédentes, **caractérisé en ce que** le deuxième élément d'outil (52, 54) peut être déplacé par rapport à la protubérance (26), transversalement à la direction longitudinale (48).

5. Instrument selon l'une des revendications précédentes, **caractérisé en ce que** le deuxième élément d'outil comprend un premier et un deuxième mors de serrage (52, 54), et en ce l'un au moins des deux mors de serrage (52, 54) porte les logements de réception (157, 159).

6. Instrument selon l'une des revendications précédentes, **caractérisé en ce que** l'instrument est conçu de façon telle que d'un pas de transport à un pas de transport suivant, la protubérance (26) peut être transportée d'au moins une distance de transport dans la direction proximale, et **en ce que** la distance de transport correspond à la distance entre dents la plus petite de la denture (152, 154) et de la denture de protubérance (160).

7. Instrument selon l'une des revendications précédentes, **caractérisé en ce que** la pluralité de dents (156, 158) de la denture (152, 154) peut être engagée, transversalement dans un logement de maintien (28), et **en ce que** la protubérance (26) présente le logement de maintien (28) destiné à recevoir au moins une dent (156, 158) de la denture (152, 154).

8. Instrument selon l'une des revendications précédentes, **caractérisé en ce que** les logements de réception (157, 159) sont d'une configuration sans arêtes.

9. Instrument selon l'une des revendications précédentes, **caractérisé en ce que** l'instrument (10) comprend un corps de base (40) et au moins un élément d'actionnement (140, 142) monté mobile sur le corps de base (40), et **en ce que** par un mouvement de l'élément d'actionnement (140, 142) par rapport au corps de base (40), une force de traction, qui s'éloigne du premier élément d'outil (34) dans la direction longitudinale (48), peut être transmise au deuxième élément d'outil (52, 54).

10. Instrument selon la revendication 9, **caractérisé en ce que** par un mouvement de l'élément d'actionnement (140, 142) par rapport au corps de base (40), une force de maintien peut être transmise au deuxième élément d'outil (52, 54) transversalement à la direction longitudinale (48).

11. Instrument selon la revendication 10, **caractérisé en ce qu'**il est prévu un organe de renvoi de force (84) destiné à dévier une force de traction agissant dans la direction longitudinale (48) pour la convertir en la force de maintien agissant transversalement à la direction longitudinale (48).

12. Instrument selon la revendication 11, **caractérisé en ce que** ledit au moins un mors de serrage (52, 54) s'appuie sur l'organe de renvoi de force (84), et peut être guidé sur celui-ci pendant un mouvement de l'organe de renvoi de force (84) dans la direction longitudinale (48).

13. Instrument selon l'une des revendications 11 ou 12, **caractérisé en ce que** ledit au moins un élément ou organe d'actionnement (140, 142) peut transmettre une force de traction à l'organe de renvoi de force (84).

14. Instrument selon l'une des revendications 11 à 13, **caractérisé en ce que** ledit au moins un mors de serrage (52, 54) s'appuie de manière élastique dans la direction longitudinale (48) sur l'organe de renvoi de force (84).

15. Instrument selon l'une des revendications 11 à 14, **caractérisé en ce que** l'organe de renvoi de force (84) s'appuie de manière élastique sur le corps de base (40).

16. Instrument selon l'une des revendications 10 ou 15, **caractérisé en ce qu'**il est prévu un dispositif de limitation de force de traction (102) pour limiter la force de traction dans la direction longitudinale (48).

17. Instrument selon la revendication 16, **caractérisé en ce qu'**à l'aide du dispositif de limitation de force de traction (102), une force initiée par ledit au moins un élément d'actionnement (140, 142), peut être transmise de manière limitée à l'organe de renvoi de force (84).

18. Instrument selon l'une des revendications 16 ou 17, **caractérisé en ce que** l'organe de renvoi de force (84) s'appuie de manière élastique sur le dispositif de limitation de force de traction (102).

19. Instrument selon l'une des revendications 16 à 18, **caractérisé en ce que** le dispositif de limitation de force de traction (102) s'appuie de manière élastique sur le corps de base (40).

20. Instrument selon l'une des revendications 10 à 19, **caractérisé en ce que** ledit au moins un mors de serrage (52, 54) est monté sur un élément de poussée et de traction (72) monté coulissant dans la direction longitudinale (48) sur le corps de base (40), et **en ce que** ledit au moins un élément d'actionnement (140, 142) peut transmettre une force de traction à l'élément de poussée et de traction (72).

21. Dispositif de fixation de plaque d'ostéosynthèse (12), qui comprend un premier élément d'appui sur l'os (14) dont fait saillie un organe de liaison (18) en forme de tige définissant une direction longitudinale (48), et un deuxième élément d'appui sur l'os (20) pouvant coulisser sur l'organe de liaison (18) en direction du premier élément d'appui sur l'os (14), dispositif
dans lequel l'organe de liaison (18) est muni de protubérances de retenue (16) rendant impossible un coulissement du deuxième élément d'appui sur l'os (20) par rapport au premier élément d'appui (14) en s'éloignant de celui-ci, en raison des protubérances de retenue (16) agissant dans cette direction,
dans lequel l'organe de liaison (18) comporte une protubérance (26) en saillie, qui présente une denture de protubérance (160) englobant au moins deux dents (30, 32),
et le dispositif de fixation de plaque d'ostéosynthèse (12) peut être appliqué à l'aide d'un instrument chirurgical (10) comprenant un premier élément d'outil (34) qui, dans une position d'application, peut être appliqué contre le deuxième élément d'appui sur l'os (20), et un deuxième élément d'outil (52, 54) qui peut être éloigné du premier élément d'outil (34), l'instrument comprenant également un dispositif de transport pour transporter pas à pas l'organe de liaison (18), à l'aide du deuxième élément d'outil (52, 54), en plusieurs pas de transport, dans une direction proximale en s'éloignant du premier élément d'outil (34) qui, dans la position d'application, est appliqué contre le deuxième élément d'appui sur l'os (20), le deuxième élément d'outil (52, 54) comportant plusieurs logements de réception (157, 159) pour la protubérance (26) en saillie de l'organe de liaison (18), et le deuxième élément d'outil (52, 54) présentant une denture (152, 154) qui comprend une pluralité de dents (156, 158), et la denture (152, 154) englobant les logements de réception (157, 159),
**caractérisé en ce que** la denture (152, 154) du deuxième élément d'outil (52, 54) présente un pas qui correspond à un multiple entier naturel d'un pas de la denture de protubérance (160).

22. Dispositif de fixation de plaque d'ostéosynthèse selon la revendication 21, **caractérisé en ce que** la protubérance (26) peut être introduite par complémentarité de formes dans les logements de réception (157, 159).

23. Dispositif de fixation de plaque d'ostéosynthèse selon la revendication 21 ou la revendication 22, **caractérisé en ce que** la protubérance (26) présente un logement de maintien (28) destiné à recevoir au moins une dent (156, 158) de la denture (152, 154).

24. Dispositif de fixation de plaque d'ostéosynthèse selon la revendication 23, **caractérisé en ce que** la denture de protubérance (160) comprend le logement de maintien (28).

25. Dispositif de fixation de plaque d'ostéosynthèse selon la revendication 23 ou la revendication 24, **caractérisé en ce que** le logement de maintien comprend une rainure annulaire (28).

26. Dispositif de fixation de plaque d'ostéosynthèse selon l'une des revendications 23 à 25, **caractérisé en ce que** la protubérance (26) est réalisée sans arêtes.

27. Dispositif de fixation de plaque d'ostéosynthèse selon l'une des revendications 23 à 26, **caractérisé en ce que** la protubérance (26) est agencée à une extrémité de l'organe de liaison (18), qui est à l'opposé de celle où se situe le premier élément d'appui sur l'os (14).

28. Dispositif de fixation de plaque d'ostéosynthèse selon l'une des revendications 23 ou 27, **caractérisé en ce que** la protubérance (26) est réalisée sous une forme annulaire.

29. Dispositif de fixation de plaque d'ostéosynthèse selon l'une des revendications 23 à 28, **caractérisé en ce que** la protubérance (26) présente un rétrécissement (28) de forme annulaire.
